# EUROPÄISCHE PATENTSCHRIFT

(11) **EP 1 312 340 B1**
(45) Veröffentlichungstag und Bekanntmachung des Hinweises auf die Patenterteilung: **31.05.2006**
(21) Anmeldenummer: 01126489.2
(22) Anmeldetag: 12.11.2001
(51) Int. Cl.: A61K 8/46, A61K 8/49, A61Q 5/10

(54) **Verfahren zum Färben von menschlichen Haaren**
Method of colouring humain hair
Procédé de teinture des cheveux humains

(43) Veröffentlichungstag der Anmeldung: 21.05.2003
(60) Teilanmeldung: 05012828.9
(73) Patentinhaber: KPSS-Kao Professional Salon Services GmbH, 64297 Darmstadt (DE)
(72) Erfinder: Möhring, Hartmut, Dr., 64342 Seeheim-Jugenheim (DE); Pratt, Dominic, Dr., 64295 Darmstadt (DE); Nöcker, Bernd, Dr., 64375 Ober-Ramstadt (DE)

(56) Entgegenhaltungen:
- DE-A- 19 731 166
- US-A- 4 102 641
- US-A- 6 086 639

## Beschreibung

Die Erfindung betrifft ein neuartiges Verfahren zum Färben von menschlichen Haaren, das langanhaltende Färbungen mit guten Farbeigenschaften wie Licht-, Wasch- und Umweltstabilität ergibt, wobei auch bei wiederholter Anwendung keine Haarschädigung auftritt.

Bei der Haarfärbung finden grundsätzlich zwei Verfahren Anwendung:

Einerseits die Färbung mit direktziehenden Farbstoffen, die zwar das Haar auch bei wiederholter Anwendung nicht schädigt, aber zu Färbungen führt, die nicht sehr langlebig sind und auch bei wiederholter Haarwäsche und durch Umwelteinflüsse verblassen, da die verwendeten direktziehenden Farbstoffe nur oberflächlich auf das Haar aufziehen.

Alternativ dazu lassen sich durch die Anwendung von Oxidationsfarbstoffzubereitung, die Oxidationsfarbstoffvorprodukte und Oxidationsmittel enthalten, dauerhafte Haarfärbungen erzielen, da dabei eine Reaktion direkt mit den Haaren stattfindet. Bei kurzfristig wiederholter Anwendung können jedoch, insbesondere bei empfindlichen Haaren, Haarschädigungen auftreten.

Die Erfindung geht von der Aufgabenstellung aus, die Nachteile beider Verfahren zu vermeiden, und eine dauerhafte Haarfärbung ohne jedwede Haarschädigung zu bewirken.

Die Lösung dieser Aufgabe besteht in einem neuen Verfahren zum Färben von menschlichen Haaren, das durch die Kombination folgender Verfahrensschritte gekennzeichnet ist:
a) Aufbringen einer Zusammensetzung mit einem pH-Wert von 2 bis 5, enthaltend mindestens einen Reaktivfarbstoff, auf menschliches Haar;
b) Einwirkenlassen zwischen etwa 5 bis 45 Minuten;
c) gegebenenfalls Spülen und Trocknen; Aufbringen einer alkalischen Zusammensetzung mit einem pH-Wert von 7,5 bis 12;
d) Einwirkenlassen für 1 bis 20 Minuten; und
e) Ausspülen bzw. Auswaschen und Trocknen des gefärbten Haares.

Dadurch wird eine dauerhafte, gegenüber Umwelteinflüssen und Haarwäschen stabile Haarfärbung erzielt, die in ihrer Wirkung einer durch Oxidationsfarbstoffe erzielbaren Haarfärbung nahekommt, ohne deren Nachteile aufzuweisen.

Reaktivfarbstoffe sind seit langem bekannt; sie weisen neben einer farbgebenden Komponente mindestens eine reaktive Gruppe im Molekül auf, die durch Reaktion mit funktionellen Gruppen des Substrats, im vorliegenden Fall das Haarkeratin, an dieses gebunden wird.
In der Regel enthalten diese Reaktivstoffe, die beispielsweise in Römpp Chemie Lexikon, 9. Aufl., S. 3805-3806, mit ausführlichen Literaturhinweisen definiert sind, auch noch hydrophile Gruppen.
Erfindungsgemäß werden bevorzugt Reaktivfarbstoffe eingesetzt, die entweder mindestens eine Vinylsulfongruppe aufweisen oder mindestens eine Sulfatoethylsulfongruppe enthalten, die Sulfat abspalten kann und in die Vinylsulfongruppe übergeht, die dann mit reaktiven Gruppen des Haarkeratins reagiert.

Die erfindungsgemäßen Reaktivfarbstoffe entsprechen den folgenden Formeln:

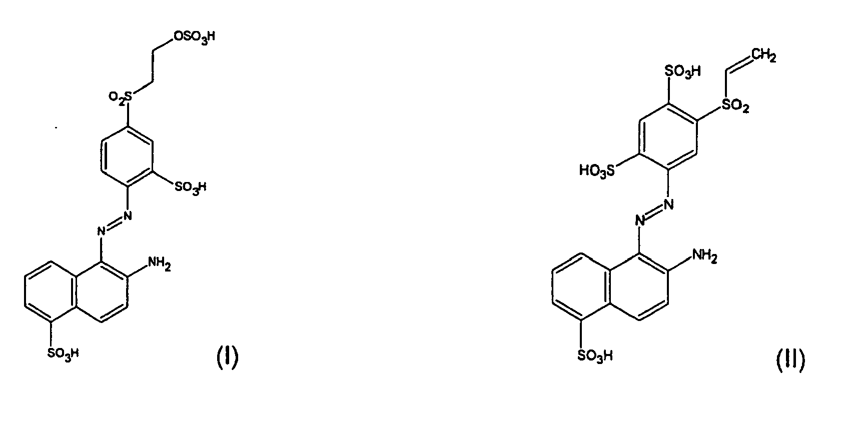

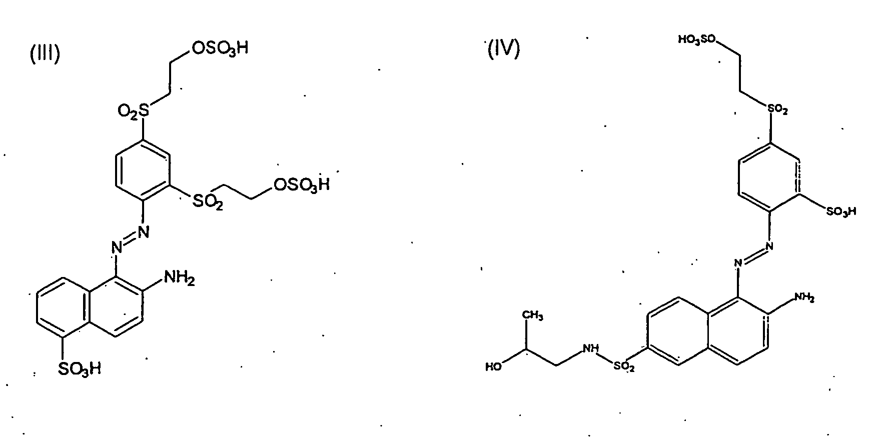

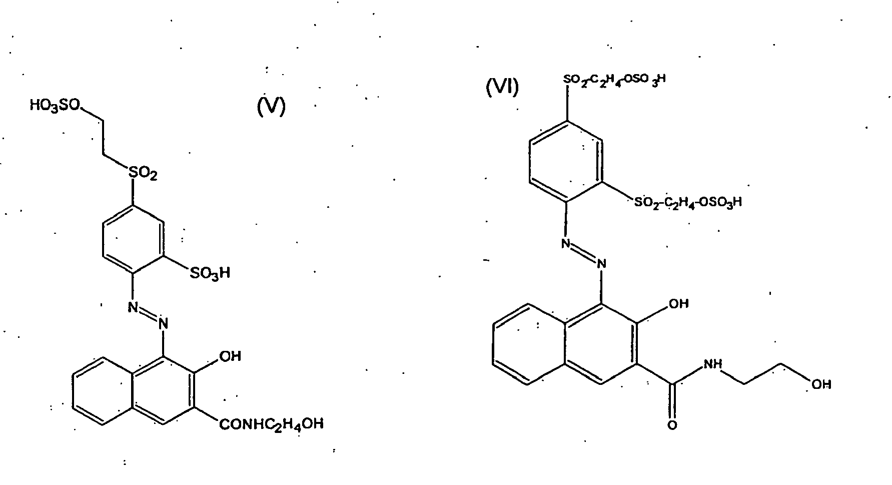

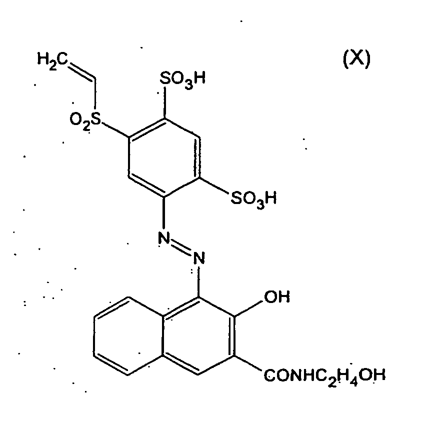

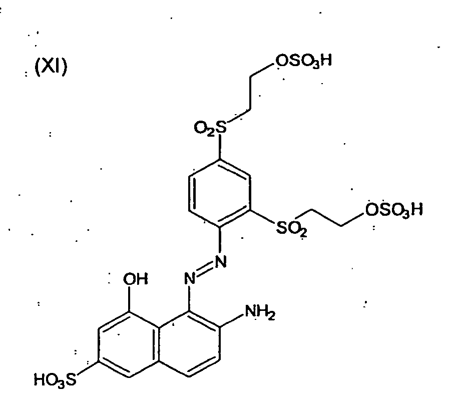

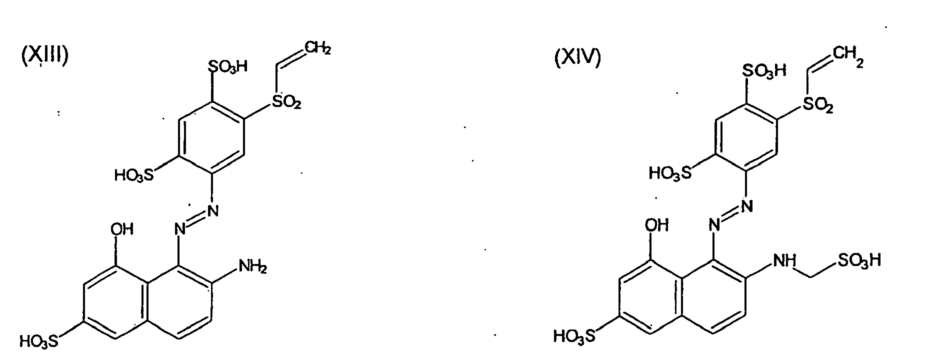

Zur Applikation können die Reaktivfarbstoffe in allen zur Haarfärbung benutzten Medien angewandt werden, insbesondere Lösungen, Emulsionen, Gelen oder (Schaum-)Aerosolen, die die in solchen Zusammensetzungen üblichen Stoffe wie oberflächenaktive Mittel, Verdickungsmittel, Riechstoffe, Stabilisatoren, Lösungsvermittler, natürliche und/oder synthetische Polymere, Komplexibildner, etc. enthalten; die Zusammensetzung dieser Trägermaterialien ist grundsätzlich bekannt und beispielsweise in der Monographie von K. Schrader, Grundlagen und Rezepturen der Kosmetika, 2. Aufl. (1989), S. 796-798, beschrieben.
Der pH-Wert der Zusammensetzung liegt zwischen 2 und 5, vorzugsweise 2, 5 bis 4, und wird auf an sich bekannte Weise eingestellt.

Die Konzentration der Reaktivfarbstoffe in der auf das Haar applizierten Zusammensetzung ist abhängig von der Struktur derselben und der erwünschten Farbintensität; sie liegt im allgemeinen bei etwa 0,01 bis etwa 5, insbesondere etwa 0,05 bis 2,5, vorzugsweise etwa 0,1 bis etwa 1 Gew.-%.
Dabei können zur Nuancierung der Färbung auch direktziehende Farbstoffe zugesetzt werden.
Die Einwirkungszeit dieser sauren Reaktivfarbstoff-Zusammensetzung liegt vorzugsweise bei etwa 10 bis 30 Minuten; sie kann durch Wärmeeinwirkung vermindert werden.
Zwischen die Verfahrensschritte b) und d), d.h. Einwirkung der sauren Reaktivfarbstoff-Zusammensetzung und Aufbringung der alkalischen Zusammensetzung, kann ein weiterer Arbeitsgang b) eingeschaltet werden, der eine zwischenzeitliche Spülung und gegebenenfalls Trocknung des Haares beinhaltet.
Die im Anschluß an den Verfahrensschritt b) und, falls fakultativ durchgeführt, c) aufzubringende alkalische Zusammensetzung weist einen pH-Wert von 7,5 bis 12, vorzugsweise etwa 8 bis 11, insbesondere etwa 8,5 bis 10 auf.

Bevorzugte Alkalisierungsmittel sind Ammoniak, Monoethanolamin sowie Alkalicarbonate und -hydrogencarbonate wie Natrium- und Kaliumcarbonat und -hydrogencarbonat.
Grundsätzlich können jedoch auch andere Alkalisierungsmittel wie Di- und Triethanolamin sowie weitere Hydroxyalkanolamine eingesetzt werden.
Die Einwirkungszeit der alkalischen Zusammensetzung auf dem Haar beträgt etwa 1 bis 20, vorzugsweise etwa 5 bis 15 Minute.
Danach wird das Haar gewaschen bzw. gespült und getrocknet.
Diese alkalische Zusammensetzung kann ebenfalls als Lösung, Emulsion, Dispersion, Gel oder (Schaum-)Aerosol vorliegen und weitere Bestandteile, z.B. pflegende oder konditionierende Substanzen, enthalten.
Diese Bestandteile sind dem Fachmann an sich bekannt und bedürfen daher keiner weiteren Erläuterungen.

Durch eine Peroxid-Behandlung kann eine weitere Aufhellung des Haares bewirkt werden.

Die folgenden Beispiele beschreiben die Erfindung im Detail:

### Beispiele

### I.

### 1. Methode

Haarsträhnen aus unbehandeltem Menschenhaar wurden angefeuchtet; anschließend wurde jeweils eine 0,1%-ige wäßrige Lösung (pH3) der unter 3. definierten Reaktivfarbstoffe gleichmäßig aufgetragen.

Nach der Einwirkung wurde mit Wasser gespült, getrocknet und anschließend eine alkalische Lösung der weiter unten definierten Zusammensetzungen aufgetragen. Nach erfolgter Einwirkung bei Raumtemperatur wurde wiederum gespült und getrocknet.
Es wurden sowohl die Farbintensität durch die bekannte Messung der ΔE-Werte als auch die Bindung des Farbstoffs an das Haar durch die Bestimmung der Stabilität der Farbstoffaufnahme mittels Extraktion durch ein Pyridin/Wasser-Gemisch im Vergleich zu nicht erfindungsgemäß behandelten Haarsträhnen, d.h. solchen, bei denen keine alkalische Nachbehandlung durchgeführt worden war, untersucht.
Die 72-stündige Extraktion durch eine 50%-ige wäßrige Pyridinlösung ist ein Maß für die Menge des Farbstoffs, der mit dem Haarkeratin reagiert hat, d.h., je mehr Farbstoff mittels Pyridin extrahiert wird, desto niedriger der Bindungsgrad und damit die Farbwirkung.

### 2. Untersuchte Zusammensetzungen:

### Saure Farbstofflösungen

### Trägerlösung:

| | |
|---|---|
| 25,0 (Gew.-%) | Propylencarbonat |
| 5,0 | Ethanol |
| 3,0 | PEG-40 Hydriertes Ricinusöl |
| 5,0 | Milchsäure |
| 0,3 | NaOH, 32%-ig |
| 0,1 | Reaktivfarbstoff |
| ad 100,0 | Wasser |

### II.

Nach der in I. beschriebenen Methode wurden weitere Reaktivfarbstoffe in Kombination mit einer alkalischen Nachbehandlung zur Haarfärbung eingesetzt und dabei der Anteil des an das Haar gebundenen Farbstoffs durch Pyridin/Wasser-Extraktion über 72 Stunden bestimmt.

Als alkalisches Behandlungsmittel wurde dabei eine Lösung aus 2,70 Gew.-% Ammoniumchlorid und 3,36 Gew.-% 25%-igem Ammoniak in 94,14 Gew.-% Wasser mit einem pH-Wert von 9,6 eingesetzt; die Einwirkungszeit betrug einheitlich 10 Minuten bei Raumtemperatur.

Die erhaltenen Ergebnisse sind in der folgenden Tabelle dokumentiert:

| Reaktivfarbstoff (Allgemeine Formel | Extrahierbarer Farbstoff aus dem Haar | Farbe |
|---|---|---|
| X | 12.% | Rot |
| XI | 13% | Violett |
| VI | 29% | Gelb |
| I | 32% | Rot |
| IV | 33% | Rot |
| V | 38% | Orange |
| II | 25% | rot |
| III | 12% | rot |

## Patentansprüche

1. Verfahren zum Färben von menschlichen Haaren, **gekennzeichnet durch** die Kombination folgender Verfahrensschritte:
a) Aufbringen einer Zusammensetzung mit einem pH-Wert von 2 bis 5, enthaltend mindestens einen Reaktivfarbstoff, ausgewählt aus 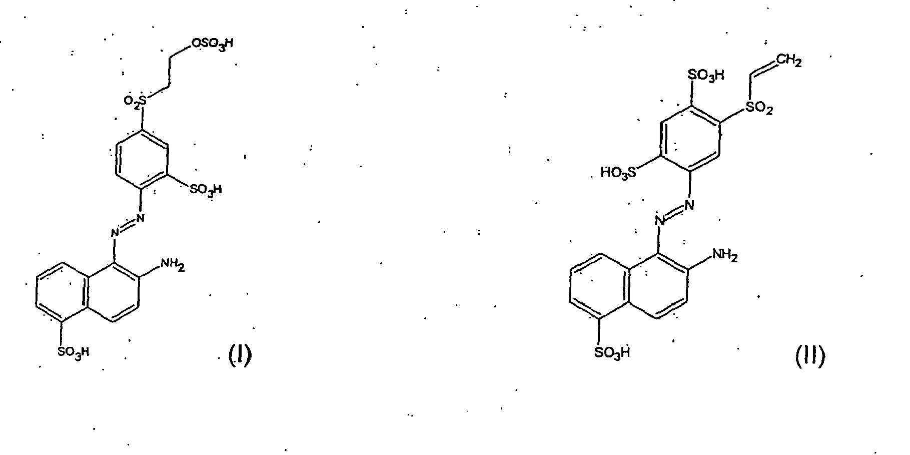 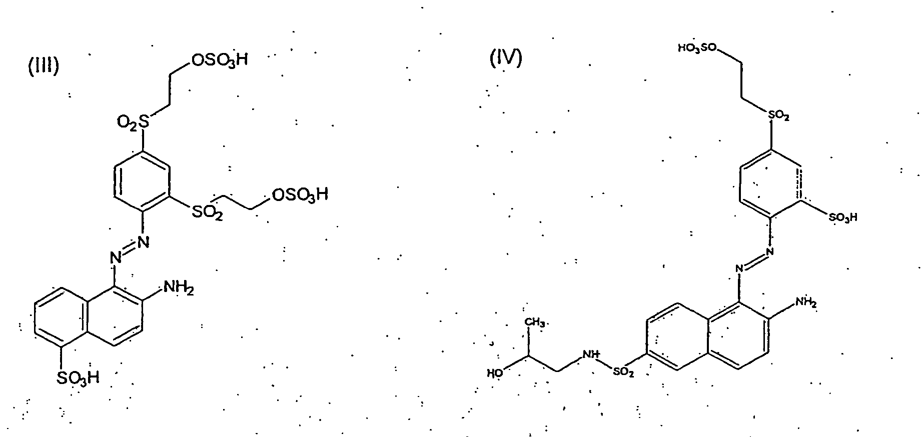 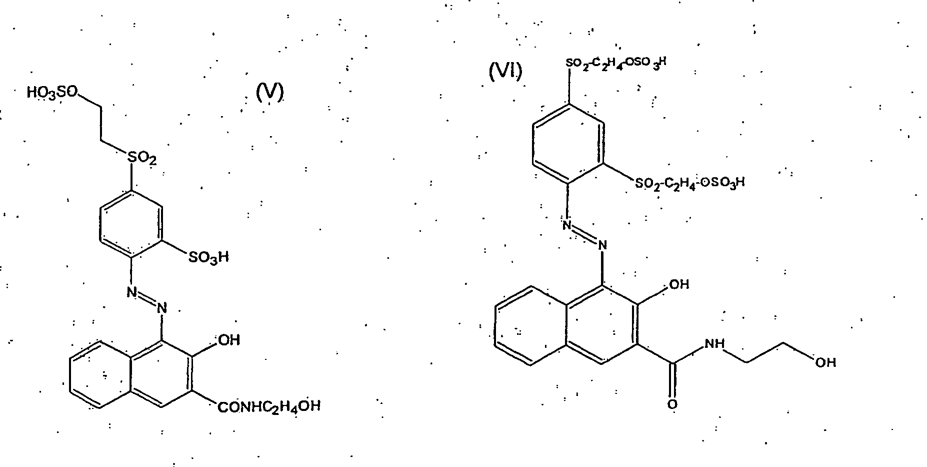 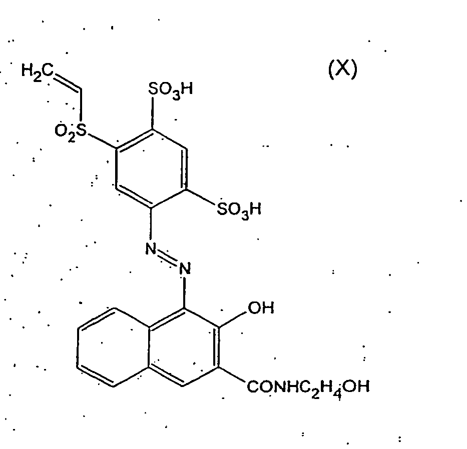 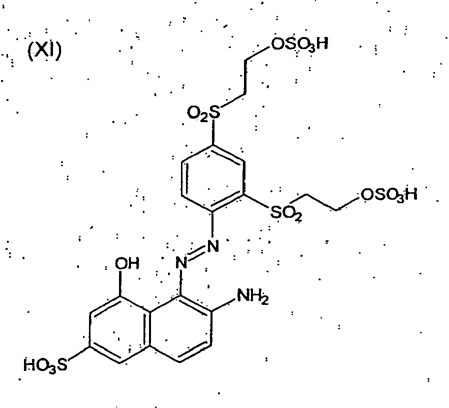 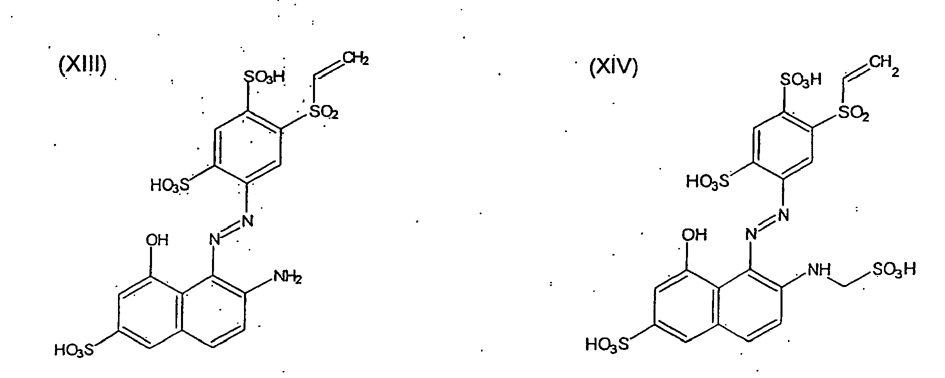 auf menschliches Haar;
b) Einwirkenlassen zwischen etwa 5 bis 45 Minuten;
c) gegebenenfalls Spülen und Trocknen;
d) Aufbringen einer alkalischen Zusammensetzung mit einem pH-Wert von 7,5 bis 12;
e) Einwirkenlassen für 1 bis 20 Minuten; und
f) Ausspülen bzw. Auswaschen und Trocknen des gefärbten Haares.

2. Verfahren nach Anspruch 1, **dadurch gekennzeichnet, daß** die in der Verfahrensstufe a) aufgebrachte Zusammensetzung mindestens einen Reaktivfarbstoff der mindestens eine Vinylsulfongruppe aufweist, enthält.

3. Verfahren nach Anspruch 1, **dadurch gekennzeichnet, daß** die in der Verfahrensstufe a) aufgebrachte Zusammensetzung mindestens einen Reaktivfarbstoff, der mindestens eine Sulfatoethylsulfongruppe aufweist, enthält.

4. Verfahren nach einem oder mehreren der Ansprüche 1 bis 3, **dadurch gekennzeichnet, daß** die in der Verfahrensstufe d) aufgebrachte alkalische Zusammensetzung einen pH-Wert von 8 bis 11 aufweist.

5. Verfahren nach einem öder mehreren der Ansprüche 1 bis 4, **dadurch gekennzeichnet, daß** die in der Verfahrensstufe d) aufgebrachte alkalische Zusammensetzung Ammoniak und/oder Mono-, Di- oder Triethanolamin enthält.

6. Verfahren nach einem oder mehreren der Ansprüche 1 bis 4, **dadurch gekennzeichnet, daß** die in der Verfahrensstufe d) aufgebrachte alkalische Zusammensetzung mindestens ein Afkaficarbohat und/oder Alkalihydrogencarbonat enthält

## Claims

1. Process for the dyeing of human hair, **characterized by** the combination of the following steps:
a) Application onto human hair of a composition, having a pH-value of 2 to 5, and comprising at least one reactive dyestuff, selected from the following formulas 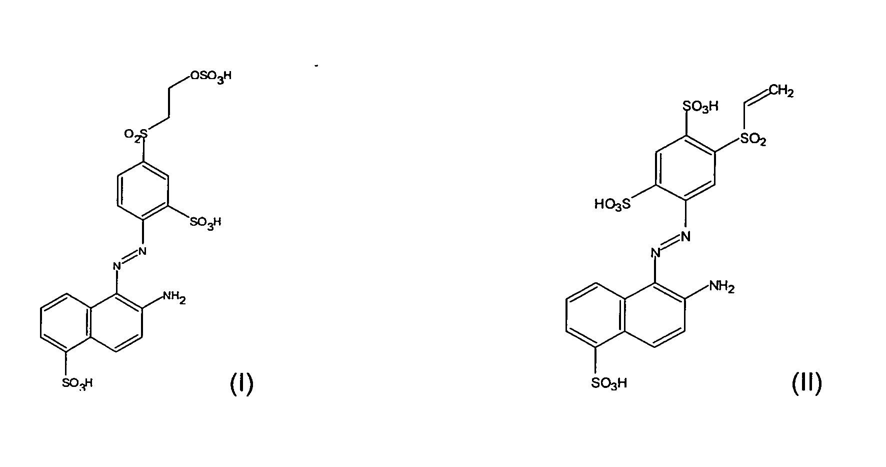 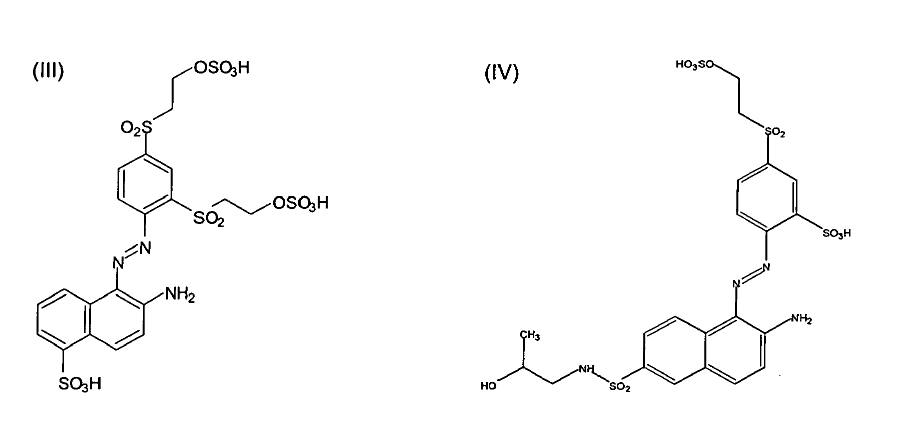 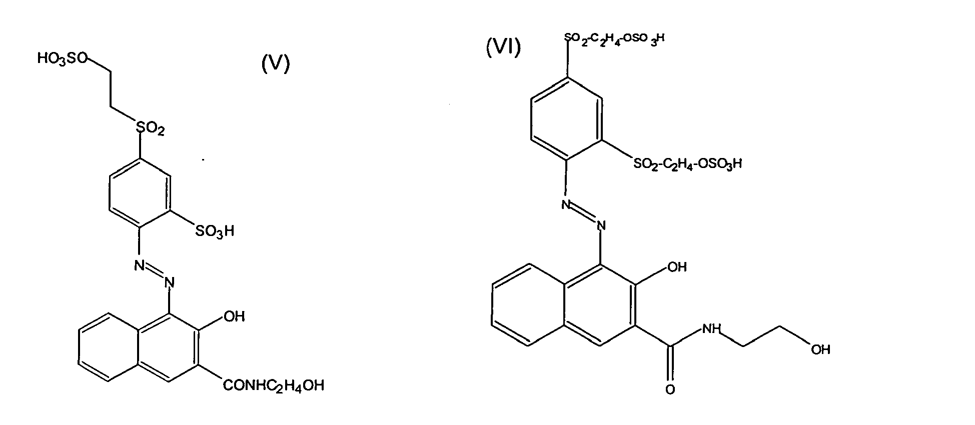 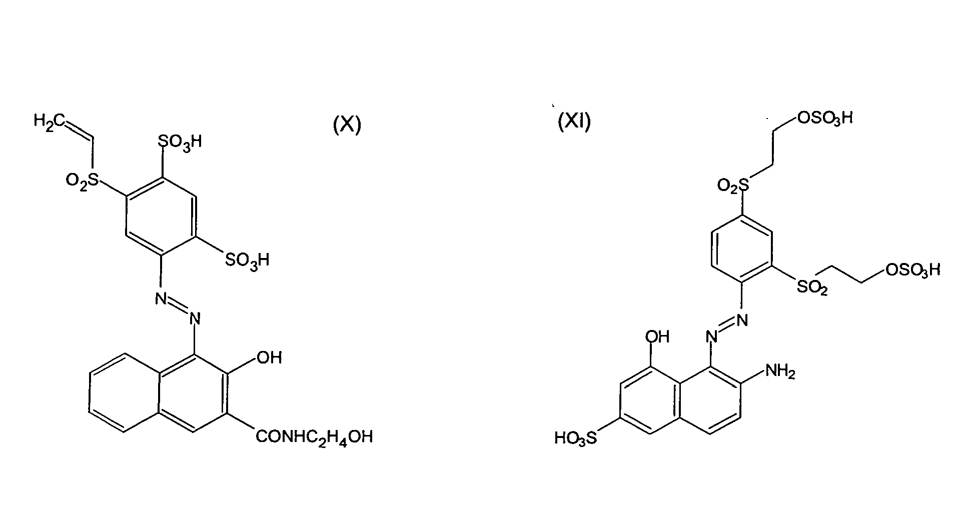 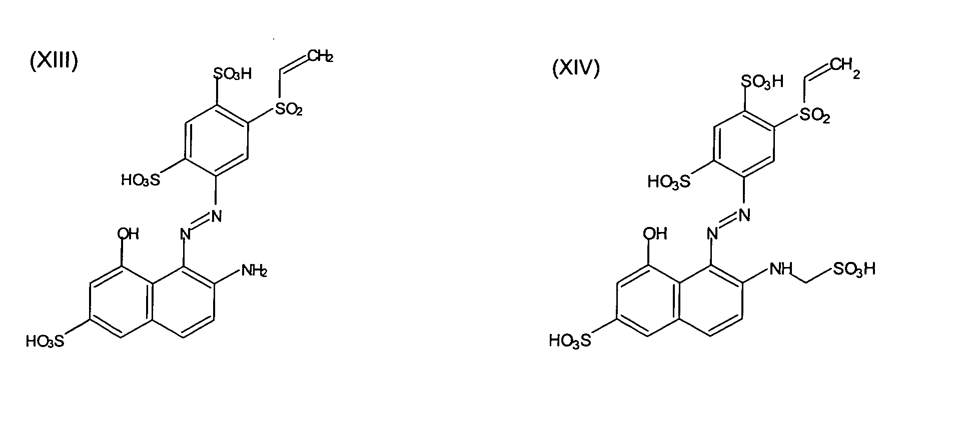
b) leaving to process between about 5 to 45 minutes;
c) optionally rinsing and drying;
d) application of an alkaline composition with a pH-value of 7.5 to 12;
e) leaving to process for 1 to 20 minutes; and
f) rinsing or shampooing and drying the colored hair.

2. Process according to claim 1, wherein the composition applied in step a) comprises at least one reactive dyestuff bearing at least one vinyl sulfone group.

3. Process according to claim 1, wherein the composition applied in step a) comprises at least one reactive dyestuff bearing at least one sulfatoethyl sulfone group.

4. Process according to one or more of claims 1 to 3, wherein the alkaline composition applied in step d) has a pH-value from 8 to 11.

5. Process according to one or more of claims 1 to 4, wherein the alkaline composition applied in step d) comprises ammonia and/or mono-, di- or triethanolamine.

6. Process according to one or more of claims 1 to 4, wherein the alkaline composition applied in step d) comprises at least one alkali carbonate and/or alkali hydrogencarbonate.

## Revendications

1. Procédé pour teindre les cheveux humains, **caractérisé par** la combinaison des étapes de procédé suivantes consistant à:
a) appliquer sur le cheveu humain une composition ayant une valeur de pH de 2 à 5, contenant au moins un colorant réactif choisi parmi 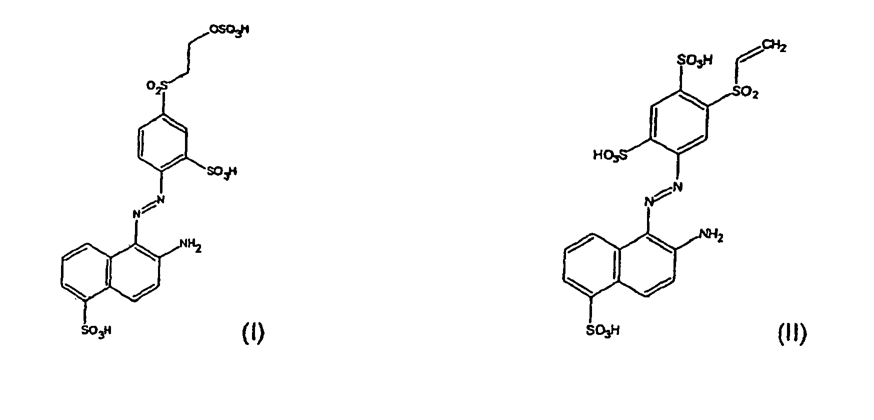 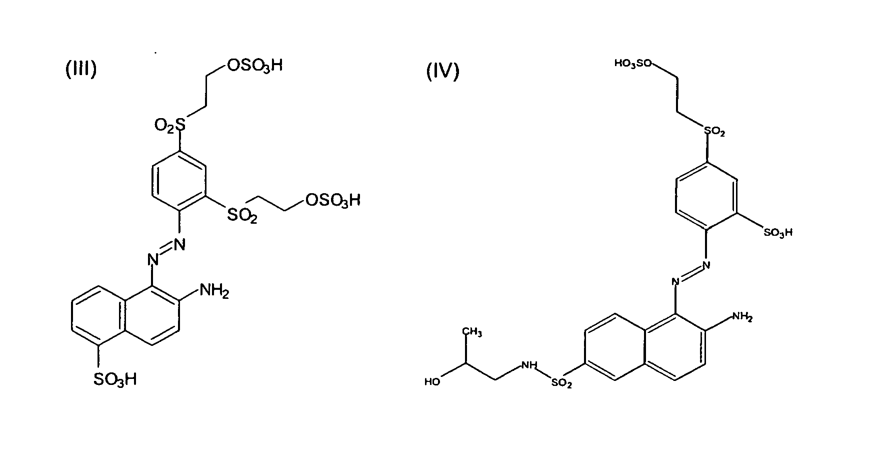 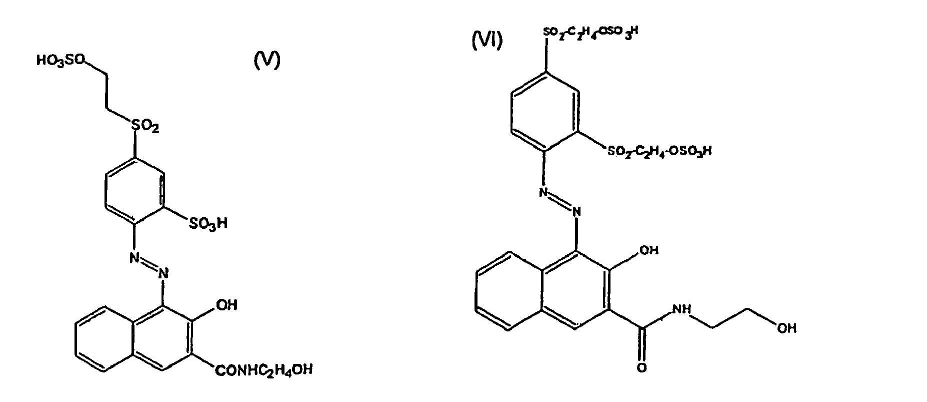 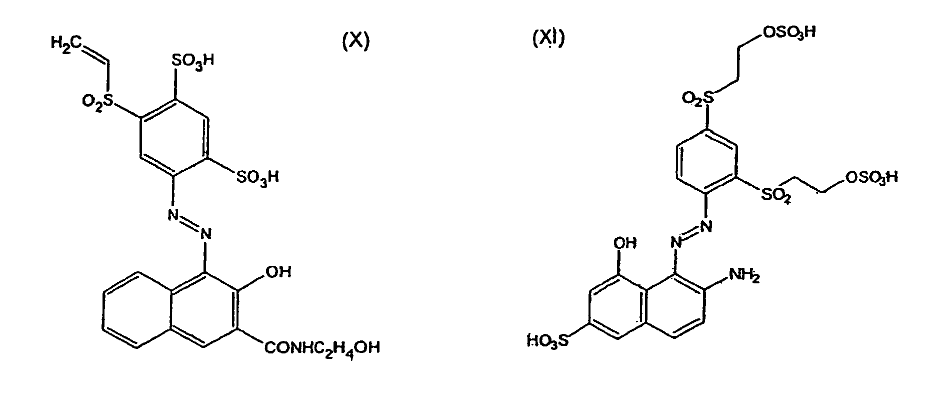 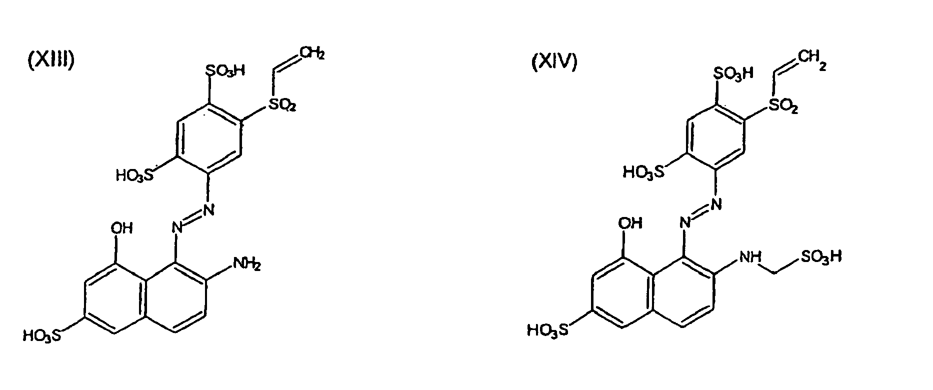
b) laisser agir pendant entre environ 5 et 45 minutes;
c) éventuellement rincer et sécher;
d) appliquer une composition alcaline ayant une valeur de pH de 7,5 à 12;
e) laisser agir pendant 1 à 20 minutes; et
f) rincer ou laver et sécher le cheveu coloré.

2. Procédé selon la revendication 1, **caractérisé en ce que** la composition appliquée dans l'étape de procédé a) contient au moins un colorant réactif qui présente au moins un groupe vinylsulfone.

3. Procédé selon la revendication 1, **caractérisé en ce que** la composition appliquée dans l'étape de procédé a) contient au moins un colorant réactif qui présente au moins un groupe sulfato-éthylsulfone.

4. Procédé selon l'une quelconque des revendications 1 à 3, **caractérisé en ce que** la composition alcaline appliquée dans l'étape de procédé d) présente une valeur de pH de 8 à 11.

5. Procédé selon l'une quelconque des revendications 1 à 4, **caractérisé en ce que** la composition alcaline appliquée dans l'étape de procédé d) contient de l'ammoniaque et/ou de la mono-, di-, tri-éthanolamine.

6. Procédé selon l'une quelconque des revendications 1 à 4, **caractérisé en ce que** la composition alcaline appliquée dans l'étape de procédé d) contient au moins un carbonate de métal alcalin et/ou un hydrogénocarbonate de métal alcalin.
